(19) Europäisches Patentamt
European Patent Office
Office européen des brevets

(11) EP 1 248 109 A1

(12) # EUROPEAN PATENT APPLICATION

(43) Date of publication:
**09.10.2002 Bulletin 2002/41**

(51) Int Cl.[7]: **G01N 33/52**

(21) Application number: **01201258.9**

(22) Date of filing: **04.04.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Rijksuniversiteit te Groningen**
**9712 CP Groningen (NL)**

(72) Inventors:
- **van Delden, Richard Andreas**
  **9642 NK Veendam (NL)**
- **Feringa, Bernard Lucas**
  **9765 EP Paterswolde (NL)**

(74) Representative: **Prins, Adrianus Willem et al**
**Vereenigde,**
**Nieuwe Parklaan 97**
**2587 BN Den Haag (NL)**

(54) **Enantiomeric excess determination using a liquid crystal material**

(57) The invention relates to a novel method for determining enantiomeric excesses of simple organic materials via an easy color test. The simple organic materials when functionalized with a designed mesogenic moiety readily induce chiral liquid crystalline (LC) phases where wavelength of reflections can be reached which reflections are in the range of the visible spectrum. The color of reflection that can be observed by eye is a measure for the enantiomeric excess. The enantiomeric excess of the relevant samples as selected by the eye can be quantified in more detail by a simple measurement of the reflection wavelength.



Printed by Jouve, 75001 PARIS (FR)

## Description

The invention relates to a new concept for fast and easy color tests for enantiomeric excess determination applicable in the field of combinatorial catalysis and as rapid test for laboratory and industrial purposes.

Since the development of combinatorial chemistry and catalysis screening the activity and selectivity of a certain catalyst in a certain reaction has always been the rate-determining step. Although in some cases screening for activity can be done by a simple color test which could in speed keep up with the speed of performing reactions (see for example J. Am. Chem. Soc., 1998, 120, 9971-9972), no such simple and instant color test has been developed for the determination of enantioselectivity yet.

In the field of combinatorial catalysis there are a number of possible ways to determine enantioselectivity (see for example Angew. Chem. Int. Ed., 2001, 40, 284-310). Most techniques, in particular those based on chromatographic methods, use separation of enantiomers and are relatively time-consuming. However, also, mainly spectroscopic techniques for determination are available. They include UV/VIS spectroscopy, fluorescence, mass spectrometry and IR thermometry.

Since all analyses mentioned above require specific techniques to be used there is a need for simple and instant enantiomeric excess (e.e.) determination methods. The present invention aims to provide such a method. It is thus an object of this invention to provide a feasible and rapid concept for determination of enantiomeric excesses of products of chiral catalytic reactions. Other objects of the invention will become clear of the more detailed description of the invention and its advantages below.

Surprisingly, it has been found that a rapid e.e. determination can be carried out by inducing a color or a color change in a liquid crystalline material through the chiral compound to be analyzed in combination with a specific derivatization agent, which will be referred to as a mesogenic unit herein.

Accordingly, the invention relates to a method for determining enantiomeric excess of a chiral analyte comprising the steps of:

a) providing a nematic liquid crystalline material;
b) derivatizing the analyte with a mesogenic unit;
c) doping said liquid crystalline material with the derivatized analyte;
d) registering a color or change in color in the liquid crystalline material; and
e) deriving the enantiomeric excess of the analyte from the color or change in color.

A method according to the invention provides a very convenient and rapid test for determining enantiomeric excess (e.e), either qualitatively or quantitatively. A color or change in color is induced that can be observed by the naked eye. This color or color change is a measure of the e.e., which can be quantified in more detail by a simple measurement of the reflection wavelength.

These characteristics of the present method imply a very significant breakthrough in the combinatorial chemistry fields. In case, for instance, many different asymmetric catalysts for a certain reaction step are to be evaluated, a huge saving in time is achieved by determining the e.e. of the reaction step in accordance with the invention. As a result, within the same period of time many more catalysts can be evaluated.

The current invention makes use of the sensitivity of liquid crystalline (LC) matrices to chiral perturbations. It is known that nematic liquid crystals can be changed to chiral nematic or cholesteric liquid crystals by doping with chiral compounds. The chirality of a cholesteric liquid crystalline material is indicated by the sign and magnitude of the cholesteric pitch.

From theory the pitch of a doped cholesteric LC phase is dependent on: 1) the concentration (c in wt.%) of the dopant; 2) the helical twisting power (HTP or β) of the dopants in and 3) the enantiomeric excess (e.e.) of the dopant, as may be presented in the following formula (1):

$$\text{pitch (p)} = (c \times \beta \times \text{e.e.})^{-1} \qquad (1)$$

The helical twisting power is an intrinsic property of any chiral molecule indicating how efficient a dopant is capable to pose a chiral packing on the liquid crystalline material. For a given substrate doped in an LC matrix at a fixed concentration, the pitch is inversely proportional to only the enantiomeric excess of the dopant and might therefore be used as a measure for the enantioselectivity of a catalytic reaction. The pitch is generally determined by the Grandjean-Cano technique, which will be known to the skilled person. This technique requires an aligned LC sample between a flat-convex lens and a flat surface and the pitch can than be obtained from distances between distinct lines as seen through a polarizing microscope. It is evident that for screening purposes this technique, if successful can hardly compete with other techniques due to the laborious sample preparation.

Cholesteric materials, however, have very interesting optical properties when the pitch of the liquid crystal comes in the region of the wavelength of visible light. Oriented LC samples (which, *e.g.*, can be oriented by a linearly rubbed polyimide covered glass plate) show a reflection at a specific wavelength $\lambda(\alpha)$, which is the wavelength of reflection with the incident light beam at an angle $\alpha$ to the normal of the surface and n is the average of the refractive indices perpendicular and parallel to the surface (2):

$$\lambda(\alpha) = n \times p \times \cos[\sin^{-1}(\sin \alpha/n)] = n \times (c \times \beta \times e.e.)^{-1} \times \cos[\sin^{-1}(\sin \alpha/n)] \quad (2)$$

Detection of the color of such a phase is easy and can be done instantly by the eye. Preparation of these oriented samples is also easily done, for instance by slow evaporation of a solution of the doped LC material on an aligned polyimide covered glass-plate. Doped colored liquid crystals therefore are useful materials for fast screening of enantiomeric excess.

The major problem to overcome in developing such a screening technique is the drawback that for normal products of enantioselective catalysis helical twisting powers are negligible (and thus no cholesteric phase can be obtained) or very small (only pitches in the range of microns can be obtained). Color induction is normally only achieved for specially designed dopants. Compounds that structurally resemble the LC material are expected to show high helical twisting powers as well as high compatibility.

In accordance with the present invention this problem has been solved by derivatizing an analyte of which the enantiomeric excess is to be determined. To this end, the invention provides a novel class of derivatization agents (mesogenic units). With these mesogenic units, a one step derivatization of simple products of catalytic reaction can be performed. Doping in nematic LC phases of the derivatization product and detection of the color as well as the maximum absorption wavelength leads to the desired information on the e.e.

The use of a mesogenic unit according to the present invention increases the compatibility and helical twisting powers of simple compounds (with no or very low helical twisting powers) to such an extent that doped liquid crystals can be obtained for which the wavelength of reflection lies in the visible region. Thus, a direct measurement of the enantiomeric excess of the dopant can be achieved. In other words, the present invention provides a simple functionalization, which enables a convenient determination of the enantiomeric excess of a chiral compound by a color test of a doped liquid crystalline matrix.

The chiral compound of which an e.e. can be determined in accordance with the invention (*i.e.* the analyte) can in principle be any chiral compound, optionally comprising multiple chiral centers. It is preferred that the analyte is a compound chosen from the group of chiral amines, chiral alcohols, and chiral carboxylic acids. It will be understood that the chiral compound should at least contain a functional group capable of binding to the mesogenic unit, of which the characteristics will be further discussed below. Typically, the compound will be a relatively simple molecule, which is a product of a test reaction for an asymmetric catalyst in a combinatorial screening method. Examples of such test reactions are well-known in the field, see for instance Angew. Chem. Int. Ed. 2001, 40, 284-310, or *Comprehensive Asymmetric Catalysis* Jacobsen, E.N., Pfaltz, A., Yamamoto, H. Eds.; Springer-Verlag; Berlin/Heidelberg 1999.

In a method according to the invention, in principle all commercially available nematic LC materials are applicable as LC phase. The term 'nematic LC material' is intended to refer to LC materials which show nematic behavior in a given temperature range. In accordance with the invention it is preferred that the LC material used is in the nematic phase at ambient conditions (room temperature and atmospheric pressure). The LC material is preferably chosen such that it has a close structural resemblance to the mesogenic unit used. Although helical twisting powers will certainly differ for a given analyte in different LC materials, this can be balanced by adjusting the concentration in such a way as to obtain a violet-colored doped LC sample for enantiomerically pure dopant.

Some examples are provided below. The indicated temperature ranges indicate at what temperature the material shows nematic crystalline behavior. Doped cholesteric phases can be induced within these ranges, but since the exact nature of the cholesteric phase is believed to be temperature dependent, it is preferred that the determination of the e.e. in accordance with the invention is performed at a constant temperature.

M15 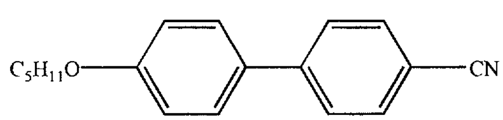 24-30°C

K15 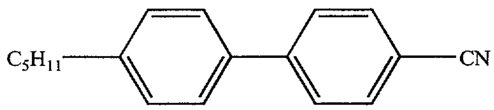 48-67°C

pCH7 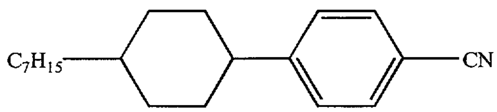 <48°C

cCH7 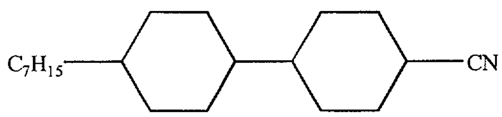 71-83°C

ZLI-389 Mixture of alkoxyphenyl and phenylbenzoates -65-35°C

K24 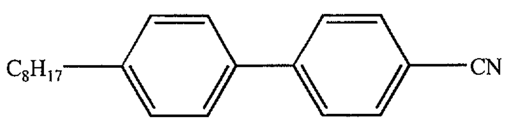 33-41°C

E7 Mixture of alkyl and alkyloxybiphenyl nitriles <58°C

M15 and K15 are commercially available from Aldrich as 4'-(pentyloxy)-4-biphenylcarbonitrile and 4'-pentyl-4-biphenylcarbonitrile, respectively. ZLI-389, K24 and E7 are commercially available from Merck. pCH7 and cCH7 are synthetic liquid crystals. Particularly preferred is the use of E7, as it is liquid crystalline at room temperature. All these materials can be used without any prior treatment.

An important aspect of the invention is the mesogenic unit. Ultimately, in a method according to the invention, the LC material is doped with the chiral compound derivatized with the mesogenic unit. This derivatization leads to a product that resembles the LC phase and gives high helical twisting powers as well as an excellent compatibility. The mesogenic unit has the following formula (I):

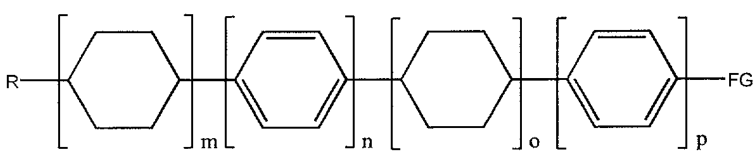 (I)

wherein:

- R is a chosen from the group of hydrogen, and substituted or unsubstituted, branched, cyclic (including aromatic moieties) or linear alkyl, alkenyl or alkynyl groups having 1 to 10 carbon atoms;
- m, n, o and p are integers of which the sum is 2 or 3; and
- FG is a functional group which can react with the chiral compound to provide the derivatized compound with which the LC material is doped.

The derivatization of a chiral compound with this mesogenic unit can be carried out via relatively mild reactions. For example, when the chiral compound is an amine, a mesogenic unit, wherein FG is an aldehyde group, can be easily attached by a simple imine formation. When the chiral compound is an alcohol, an esterification reaction with a mesogenic unit in which FG is an acid chloride gives the desired derivatized chiral compound in a convenient manner.

One can also think of reacting amino acids or chiral diamines with the above mentioned aldehyde or chiral diols with the above mentioned acid chloride and the concept can be extended to a whole range of different compounds by changing the functional group to for example a hydroxyl of halogen. Table 1 provides some more detailed insight how FG is to be chosen in order to achieve an easy, mild and simple derivatization of the chiral compound with the mesogenic unit. As used in Table 1, R' denotes a substituent chosen from the group of substituted or unsubstituted, branched, cyclic (including aromatic moieties), or linear alkyl, alkenyl or alkynyl groups having from 1 to 10 carbon atoms.

Table 1

| Functional group in chiral compound | FG |
|---|---|
| amine, diol | COH/COR' |
| alcohol, amine, thiol | COOH/COCl/COOR' |
| boronic acid | Cl/Br/I |
| halide | MgBr/Li |
| carboxylic acid, alcohol, halide | OH |
| aldehyde, ketone | $NH_2$ |
| alcohol, amine, carboxylic acid, thiol | NCO |
| halide | $B(OH)_2$ |

Accordingly, it is preferred that FG is chosen from the group of aldehydes (COH), ketones (COR', wherein R' is chosen from the group of substituted or unsubstituted, branched, cyclic (including aromatic moieties), or linear alkyl, alkenyl or alkynyl groups having from 1 to 10 carbon atoms), carboxylic acids (COOH) or derivatives thereof such as acid chlorides (COCl) or esters (COOR', wherein R' is chosen from the group of substituted or unsubstituted, branched, cyclic (including aromatic moieties), or linear alkyl, alkenyl or alkynyl groups having from 1 to 10 carbon atoms), halides (Cl, Br, I), metals (MgBr, Li), alcohols (OH), amines, such as primary amines ($NH_2$), isocyanates (NCO) and boronic acids ($B(OH)_2$).

The conditions used for carrying out the derivatization of the chiral compound with the mesogenic unit will of course strongly depend on the type of reaction that is to be carried out. As mentioned before, it is one of the advantages of the invention that FG in the mesogenic unit can be chosen such that a very simple and convenient reaction can be performed for derivatizing the chiral compound. Accordingly, the conditions and other parameters for these reactions are within the normal expertise of the person skilled in the field of organic chemistry. Furthermore, the derivatization reactions can easily be automated in a combinatorial screening protocol.

After derivatization, the analyte-mesogenic unit molecule (dopant) will be used for doping the liquid crystalline material. The step of doping may comprise a simple mixing step of the dopant with the LC material.

The doping of the LC material with the dopant will result in a color or color change of the LC material. For a very rapid test, this color or color change may be compared with a pre-calibrated color chart to give a qualitative result. It is also possible to measure the wavelength of the color or the difference in wavelengths of the color change in order to obtain a more accurate result. The wavelength can be measured using standard spectroscopic techniques either in reflection or in transmission. The obtained wavelength or difference in wavelengths may be compared to a predetermined calibration curve to arrive at a result for the enantiomeric excess.

The present method can be exploited for fully automated enantiomeric excess determination in combinatorial catalysis with large arrays of compounds analyzed simultaneously. Fully automated preparation of aligned cholesteric films is already applied in the field of liquid crystal display technology and combining these techniques together with the present invention may result in a fully automated fast screening method for enantioselective catalysts in any catalytic reaction.

The invention will now be further elucidated by the following non-restrictive examples:

Example 1: Synthesis of the starting materials

a) aldehyde

Under a nitrogen atmosphere 13.7 g (74 mmol) of p-bromobenzaldehyde was dissolved in 200 ml of toluene. To this solution was added 1.73 g (1.5 mmol) $Pd(PPh_3)_4$ and 50 ml of a 2M $Na_2CO_3$ solution. Successively, 13.5 g (89 mmol) of p-methoxyphenyl boronic acid in 20 ml of toluene was added dropwise and the mixture was refluxed over night. After the reaction was complete the remaining boronic acid was oxidized by careful addition of 0.8 ml of 30% hydrogen peroxide solution after cooling of the reaction mixture. The mixture was then allowed to stir for one hour. The product was extracted with ether, washed with brine and dried on sodium sulfate. The crude product can be purified

by chromatography on a chromatotron ($SiO_2$; n-hexane/dichloromethane 2/1; $R_f$ = 0.2). Purified yield was 9.54 g (45 mmol; 61%) of white 4'-methoxy[1,1'-biphenyl]-4-carbaldehyde.

b) acid chloride

Under a nitrogen atmosphere 3.7 g (20 mmol) of p-bromobenzoic acid was dissolved in 75 ml of toluene. To this solution was added 530 mg (0.5 mmol) $Pd(PPh_3)_4$ and 15 ml of a 2M $Na_2CO_3$ solution. Successively, 3.69 g (24 mmol) of p-methoxyphenyl boronic acid in 20 ml of toluene was added dropwise and the mixture was refluxed overnight. After the reaction was complete the remaining boronic acid was oxidized by careful addition of 0.3 ml of 30% hydrogen peroxide solution after cooling of the reaction mixture. The mixture was then allowed to stir for one hour. The reaction mixture was poured onto water and the solid acid (2.88 g; 12.63 mmol; 63%) was collected by filtration and due to its low solubility in any organic solvent used without purification and characterization.

To 2.28 g (10 mmol) of 4'-methoxy[1,1'-biphenyl]-4-carboxylic acid an excess of 15 ml of $SOCl_2$ and a few drops of DMF were added. The obtained solution was refluxed for 3 hours and the solvent was evaporated. The obtained solid material was recrystallized from pet. ether 80/110 yielding 1.61 g (6.5 mmol; 65%) of white crystalline 4'-methoxy [1,1'-biphenyl]-4-carboxylic acid chloride.

Example 2: Functionalization of α-Phenylethylamine with mesogenic unit

106 mg (0.5 mmol) of 4'-methoxy[1,1'-biphenyl]-4-carbaldehyde was dissolved in 5 ml of dichloromethane. 250 mg of magnesium sulfate and 64 µl (60.2 g; 0.5 mmol) of α-phenylethylamine were subsequently added and the mixture was stirred at room temperature over night. After filtration and removal of the solvent the desired imine was obtained in quantitative yield. Both optical pure as well as racemic material were obtained in this way by starting with either racemic or pure (S) - α-phenyl ethyl amine.

Example 3: Functionalization of 1-phenylpropanol with mesogenic unit

345 mg (1.4 mmol) of 4'-methoxy[1,1'-biphenyl]-4-carboxylic acid chloride in 1 ml (5 fold excess) of 1-phenylpropanol was refluxed over night and the excess 1-phenylpropanol was removed by Kugelrohr destillation to yield a quantitative amount of 1-phenylpropyl 4'-methoxy[1,1'-biphenyl]-4-carboxylate. Optical pure material was obtained either by using optically active 1-phenylpropanol as starting material or by preparative chiral HPLC separation (Chiralcel OD; n-heptane/isopropanol 90/10) of the obtained racemic product.

Example 4: Preparation of films of aligned cholesteric material

A glass surface (typically 6.25 $cm^2$) was spin-coated with commercially available polyimide AL1051 (JSR; Japan) and the coated samples were allowed to polymerize at 170°C in vacuo for 3 hours. The surface was then linearly rubbed with a velvet cloth to induce a parallel aligned LC phase. The liquid crystalline material doped with the appropriate amount of dopant (see Examples 5 and 6) was dissolved in toluene (typically 1 ml of toluene per 5 mg sample) and slowly poured onto the aligned surface. After slow evaporation of the toluene at room temperature an aligned LC film was obtained. When the pitch of the cholesteric material is in the range of the wavelength of visible light these films immediately show colored reflections.

Example 5: Determination of suitable concentration of chiral imine

Commercially available liquid crystalline material E7 was chosen as the LC material because of the fact that it is liquid crystalline at room temperature and it is structurally similar to the imine to be analyzed. The helical twisting power of the imine was determined using a Grandjean-Cano technique technique (Mol. Cryst. Liq. Cryst. 1977, 41, 245-249) to be 21.4 ± 0.9 $\mu m^{-1}$. From this value the concentration of enantiomerically pure dopant needed to obtain a violet LC film, that is an LC film with a reflection wavelength of about 370 nm, can be estimated (using Formula 2). When the color of the LC phase doped with enantiomerically pure dopant (ee = 1) is observed perpendicular to the surface (α = 0°), this formula simplifies to:

$$\lambda(0) = n \times \cdot(c \times \beta)^{-1} \quad (3)$$

For the estimation the average refractive index (n = 1.6287) of the undoped material was taken giving for λ(0) = 0.37 a concentration (c) of 20.6 weight %. Using this estimated value for the concentration LC samples doped with 19, 20, 21 and 22 weight % of enantiomerically pure dopant were prepared and their color analyzed. A nice violet color was observed for the 19 weight % dopant while the other samples were colorless or only had a violet glow and this concentration was determined to be the appropriate one for use of this doped LC phase as a color indicator of chirality.

Example 6: Wavelengths of reflection for imine

Samples of enantiomeric excess of 50, 60, 70, 80, 90 and 100% of the chiral imine (Example 2) were prepared by mixing enantiomerically pure material with racemic material in the appropriate ratios. Starting with amines with the same enantiomeric excesses would be an alternative. The imine was mixed with E7 to obtain samples of 18.9 w% (typically 1.165 mg of imine in 5.000 mg of E7) and films of these mixtures were prepared according to Example 4. The wavelengths of reflected light were measured at an angle of 45°.

Table 2

| Enantiomeric Excess (%) | wavelength of reflection (nm) |
|---|---|
| 100 | 363 |
| 90 | 381 |
| 80 | 410 |
| 70 | 472 |
| 60 | 541 |
| 50 | 628 |

Example 7: Helical twisting powers and wavelengths of reflection for ester

Analogous to Example 5, the helical twisting power of the ester to be analyzed in E7 was determined to be 28.8 ± 0.7 $\mu m^{-1}$. The appropriate concentration was determined to be approximately 15 weight %. Samples of enantiomeric excess of 50, 60, 70, 80, 90 and 100% of the chiral ester (Example 3) were prepared by mixing enantiomerically pure material with racemic material in the appropriate ratios comparable to Example 6. The ester was mixed E7 to obtain samples of 14.8 w% (typically 0.869 mg of imine in 5.000 mg of E7) and films of the mixtures were prepared according to Example 4. The wavelengths of reflected light were measured at an angle of 45°:

Table 3

| Enantiomeric Excess (%) | wavelength of reflection (nm) |
|---|---|
| 100 | 359 |
| 90 | 412 |
| 80 | 461 |
| 70 | 531 |
| 60 | 586 |
| 50 | 662 |

Combining the data from Examples 5 and 6 gives a graphical presentation of the dependence of wavelength of reflection ($\lambda$ (45°)) on enantiomeric excess. With such a graph in hand one can by measuring wavelength of reflection of a doped LC material readily obtain the enantiomeric excess. Where the closed squares represent the imine (Example 5) and the open squares represent the ester (current example). When analyzing a sample of either the imine or the ester with unknown enantiomeric excess these curves can be used as calibration curves.

Example 7: Qualitative determination

Although all the experiments presented (Examples 4 and 5) above still require a measurement of the wavelength of the reflected light which is time-consuming from the point of view of screening the enantioselectivity of combinatorial catalysis processes all the colors can of course be observed instantly by eye. Table 4 below shows the colors of the doped LC phases (prepared as in Example 4 and 5) with different enantiopurity taken from a simple photograph for the investigated substrates.

Table 4

| Enantiomeric Excess | Color of E7 doped with 18.9 weight % of imine | Color of E7 doped with 14.8 weight % of ester |
|---|---|---|
| 100 | violet | violet |
| 90 | violet | violet |
| 80 | blue | blue/green |
| 70 | green | green/yellow |
| 60 | orange | orange |
| 50 | red | colorless/red |

**Claims**

**1.** A method for determining enantiomeric excess of a chiral analyte comprising the steps of:

a) providing a nematic liquid crystalline material;
b) derivatizing the analyte with a mesogenic unit;
c) doping said liquid crystalline material with the derivatized analyte;
d) registering a color or change in color in the liquid crystalline material; and
e) deriving the enantiomeric excess of the analyte from the color or change in color.

**2.** A method according to claim 1, wherein the chiral analyte is a compound chosen from the group of chiral amines, chiral alcohols, and chiral carboxylic acids.

**3.** A method according to claim 1 or 2, wherein the nematic liquid crystalline material is chosen from the group of M15, K15, pCH7, cCH7, ZLI-389, K24, and E7.

**4.** A method according to any of the preceding claims, wherein the mesogenic unit has the formula (I):

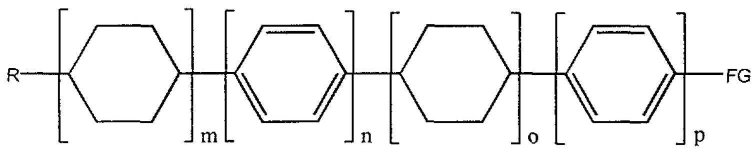

(I)

wherein:

- R is a chosen from the group of hydrogen, and substituted or unsubstituted, branched, cyclic (including aromatic moieties) or linear alkyl, alkenyl or alkynyl groups having 1 to 10 carbon atoms;
- m, n, o and p are integers of which the sum is 2 or 3; and
- FG is a functional group which can react with the chiral compound to provide the derivatized compound with which the LC material is doped.

**5.** A method according to claim 4, wherein FG is chosen from the group of aldehydes (COH), ketones (COR', wherein R' is chosen from the group of substituted or unsubstituted, branched, cyclic (including aromatic moieties), or linear alkyl, alkenyl or alkynyl groups having from 1 to 10 carbon atoms), carboxylic acids (COOH) or derivatives thereof such as acid chlorides (COCl) or esters (COOR', wherein R' is chosen from the group of substituted or unsubstituted, branched, cyclic (including aromatic moieties), or linear alkyl, alkenyl or alkynyl groups having from 1 to 10 carbon atoms), halides (Cl, Br, I), metals (MgBr, Li), alcohols (OH), amines, such as primary amines ($NH_2$), isocyanates (NCO) and boronic acids ($B(OH)_2$).

**6.** A method according to any of the preceding claims, wherein the color or change in color is registered by use of a spectroscopic technique.

**7.** A mesogenic unit having the formula (I):

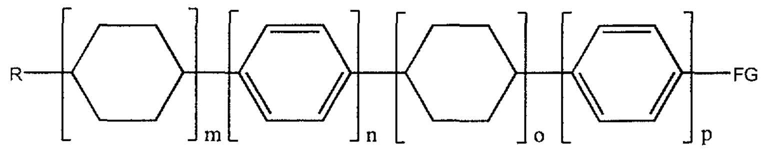

(I)

wherein:

- R is a chosen from the group of hydrogen, and substituted or unsubstituted, branched, cyclic (including aromatic moieties) or linear alkyl, alkenyl or alkynyl groups having 1 to 10 carbon atoms;
- m, n, o and p are integers of which the sum is 2 or 3; and
- FG is a functional group chosen from the group of aldehydes (COH), ketones (COR', wherein R' is chosen from the group of substituted or unsubstituted, branched, cyclic (including aromatic moieties), or linear alkyl, alkenyl or alkynyl groups having from 1 to 10 carbon atoms), carboxylic acids (COOH) or derivatives thereof such as acid chlorides (COCl) or esters (COOR', wherein R' is chosen from the group of substituted or unsubstituted, branched, cyclic (including aromatic moieties), or linear alkyl, alkenyl or alkynyl groups having from 1 to 10 carbon atoms), halides (Cl, Br, I), metals (MgBr, Li), alcohols (OH), amines, such as primary amines ($NH_2$), isocyanates (NCO) and boronic acids ($B(OH)_2$).

**8.** A chiral analyte derivatized with a mesogenic unit according to claim 7.

**9.** An analyte according to claim 8, wherein the analyte is a compound chosen from the group of chiral amines, chiral alcohols, and chiral carboxylic acids.

**10.** Use of a derivatized analyte according to claim 8 or 9 for inducing a change in a liquid crystalline material from a nematic phase to a cholesteric phase.

**11.** A method for performing combinatorial chemistry or high throughput screening comprising a determination of enantiomeric excess by a method according to any of the claims 1-6.

**12.** A method according to claim 11, wherein asymmetric catalysts are evaluated.

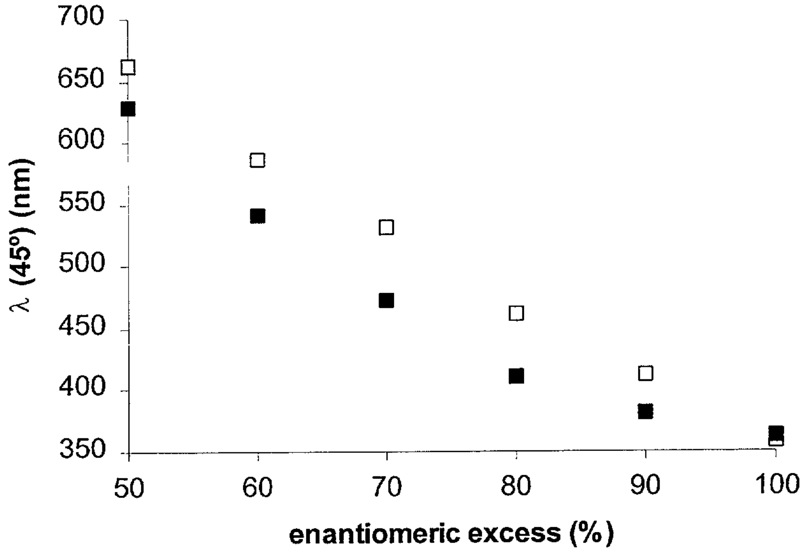

700
650
600
550
500
450
400
350
λ (45°) (nm)
50
60
70
80
90
100
enantiomeric excess (%)


Figure 1

European Patent Office

# EUROPEAN SEARCH REPORT

Application Number
EP 01 20 1258

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| X | JANINI G M ET AL: "HIGH-TEMPERATURE NEMATIC LIQUID CRYSTAL FOR GAS-LIQUID CHROMATOGRAPHY"<br>ANALYTICAL CHEMISTRY, AMERICAN CHEMICAL SOCIETY. COLUMBUS, US,<br>vol. 52, 1 December 1980 (1980-12-01), pages 2417-2420, XP000608598<br>ISSN: 0003-2700<br>* abstract *<br>* table 1 *<br>--- | 7-10 | G01N33/52 |
| X | G SOLLADIE, R ZIMMERMANN: "A New Class of Chiral Smectic Crystals: Substituted Biphenylylcyclohexylideneethanones Having an Axial Chirality"<br>JOURNAL OF ORGANIC CHEMISTRY,<br>vol. 50, no. 21, 1985, pages 4062-4068, XP002190807<br>* the whole document *<br>--- | 7-10 | |
| A | KORTE, ERNST H. ET AL: "A new method for the study of molecular chirality: infrared rotatory dispersion of induced cholesteric solutions"<br>J. CHEM. RES. (S) (1978), (7), 236-7 , XP008000599<br>* the whole document *<br>--- | 1 | TECHNICAL FIELDS SEARCHED (Int.Cl.7)<br>G01N |
| D,A | REETZ M T: "COMBINATORIAL AND EVOLUTION-BASED METHODS IN THE CREATION OF ENANTIOSELECTIVE CATALYSTS"<br>ANGEWANDTE CHEMIE. INTERNATIONAL EDITION, VERLAG CHEMIE. WEINHEIM, DE,<br>vol. 40, no. 2,<br>19 January 2001 (2001-01-19), pages 285-310, XP000992015<br>ISSN: 0570-0833<br>* the whole document *<br>---<br>-/-- | 1 | |

The present search report has been drawn up for all claims

| Place of search | Date of completion of the search | Examiner |
|---|---|---|
| THE HAGUE | 20 February 2002 | Hart-Davis, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 01 20 1258

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | SARFATI, MURIEL ET AL: "Theoretical and experimental aspects of enantiomeric differentiation using natural abundance multinuclear NMR spectroscopy in chiral polypeptide liquid crystals" CHEM. COMMUN. (CAMBRIDGE) (2000), (21), 2069-2081 , XP002190808 * the whole document * | 1 | |
| T | VAN DELDEN, RICHARD A. ET AL: "Colour indicator for enantiomeric excess and assignment of the configuration of the major enantiomer of an amino acid ester" CHEMICAL COMMUNICATIONS (CAMBRIDGE, UNITED KINGDOM) (2002), (2), 174-175 , XP002190809 * the whole document * | 1-12 | |
| T | VAN DELDEN, RICHARD A. ET AL: "Color indicators of molecular chirality based on doped liquid crystals" ANGEWANDTE CHEMIE, INTERNATIONAL EDITION (2001), 40(17), 3198-3200 , XP002190810 * the whole document * | 1-12 | TECHNICAL FIELDS SEARCHED (Int.Cl.7) |

The present search report has been drawn up for all claims

| Place of search | Date of completion of the search | Examiner |
|---|---|---|
| THE HAGUE | 20 February 2002 | Hart-Davis, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)